# EUROPEAN PATENT APPLICATION

(11) **EP 3 495 480 A1**
(43) Date of publication of application: **12.06.2019**
(21) Application number: 16911607.6
(22) Date of filing: 03.08.2016
(51) Int. Cl.: C12N 15/00

(54) **METHOD FOR PREPARING PEPTIDE FRAGMENTS, METHOD FOR PREPARING PROTEASE TO BE USED THEREIN, AND KIT FOR PREPARING PEPTIDE FRAGMENTS**

(71) Applicant: Shimadzu Corporation, Kyoto-shi, Kyoto 604-8511 (JP)
(72) Inventor: SHIMADA, Takashi, Kyoto-shi Kyoto 604-8511 (JP); IWAMOTO, Noriko, Kyoto-shi Kyoto 604-8511 (JP); AOKI, Chikage, Kyoto-shi Kyoto 604-8511 (JP)
(74) Representative: Mewburn Ellis LLP
(86) International application number: PCT/JP2016/072787
(87) International publication number: WO 2018/025346

(57) **Abstract**

The method according to the present invention for preparing peptide fragments comprises bringing an antibody, which includes a Fc domain immobilized in pores of a porous body, into contact with a protease immobilized on surface of microparticles. Thus, the Fab domain of the antibody is site-specifically cleaved by the protease and a sample containing Fab domain-derived peptide fragments at a high concentration can be obtained. The protease to be used in the present invention is an animal-derived trypsin contaminated with chymotrypsin wherein the chymotrypsin is inactivated and the trypsin is chemically modified at amino group in lysine residues.

## Description

### TECHNICAL FIELD

The present invention relates to a method for preparing peptide fragments by site-specifically cleaving an antibody using protease, a method for preparing protease used for the above, and a kit for preparing peptide fragments.

### BACKGROUND ART

Demand for antibody drugs has rapidly grown and quantitation of the concentration of an antibody in blood has become important in clinical practice. Heretofore, the accurate measurement of the concentration of an antibody drug in blood after administration has not been regarded as important. However, measurement of the concentration of an antibody in blood has become essential also in clinical trials or the like for antibody drugs since it was found that, in clinical trials performed to expand the application of trastuzumab (trade name: Herceptin) to gastric cancer, there were significant differences in the concentration of trastuzumab in blood and overall survival. For example, identification of an antibody drug or quantitation of the concentration of an antibody drug in blood is required also in quality control, such as pharmacokinetic determination, confirmation of identity with original drugs in clinical trials for generic drugs, or the like.

An antibody has one Fc (Fragment, crystallizable) domain comprising heavy chains and two Fab (Fragment, antigen binding) domains comprising a heavy chain and a light chain. An N-terminal part of each Fab domain diversely changes in its amino acid sequence so as to be able to bind to various antigens. This region is called a variable region (V region), and the specificity (i.e., specific bindability to an antigen) of an antibody is determined by the combination of amino acid sequences of the V region. The light chain and the heavy chain each have three complementarity determining regions (CDRs) in the V region. The CDR is a region characterizing the antibody: by identifying the amino acid sequence of a CDR of an antibody, the antibody can be identified.

With the development of proteomics, a technique has been developed in which proteins can be comprehensively detected and quantitated by mass spectrometry. In recent years, analysis of a macrobiomolecule, such as an antibody, has also become possible. In mass spectrometry, a molecule to be measured needs to be ionized. Therefore, it is often difficult for a macrobiomolecule, such as an antibody, to be directly analyzed by mass spectrometry. For this reason, a method is adopted in which a protein is cleaved (fragmented) by proteolysis, a peptide fragment having an amino acid sequence specific to the protein to be analyzed is selected from peptide fragments, and the selected peptide fragment is detected and quantitated by a mass spectrometer.

Since a CDR-derived peptide fragment of an antibody has an amino acid sequence specific to the antibody, quantitation of a specific antibody is possible by detecting and quantitating a CDR-derived peptide fragment by mass spectrometry. As a method for selectively producing a CDR-derived peptide fragment from an antibody, a technique has been developed in which an antibody is site-specifically cleaved by reaction of the antibody immobilized in pores of a porous body with protease immobilized on the surfaces of nano-particles to produce peptide fragments (nano-Surface and Molecular-Orientation Limited proteolysis: nSMOL method) (Patent Document 1, for example).

In the nSMOL method, by binding the Fc domain of an antibody into pores of a porous body, access of protease to the Fc domain is limited, to allow the Fab domains including CDRs to site-specifically react with protease. Therefore, in a sample after the reaction with the protease, CDR-derived peptide fragments of the antibody are contained at high concentration relative to the total amount of the produced peptides, so that highly accurate quantitative analysis is possible.

It is reported that high-accuracy quantitative analysis is possible by the nSMOL method for various antibody drugs. Concretely, reported are examples in which the concentrations of humanized antibodies such as trastuzumab (Non-Patent Documents 1 and 2), bevacizumab (Non-Patent Documents 1 and 3), and nivolumab (Non-Patent Document 4) and chimeric antibodies such as cetuximab (Non-Patent Document 5) and rituximab (Non-Patent Document 6) were quantitated by the nSMOL method.

In all of the above patent documents and non-patent documents, trypsin is used as the protease for fragmenting antibodies. Trypsin is a serine protease having a molecular weight of about 23 kDa mainly produced in the pancreas, and cleaves a carboxy group at the C-terminal of a basic amino acid (lysine and arginine) by hydrolysis. Trypsin, which is extremely high in substrate specificity, has high usability as the protease for producing peptide fragments for mass spectrometry, and therefore is used for structural analysis of many proteins.

Trypsin extracted from an animal's pancreas has chymotrypsin mixed therein. The substrate specificity of chymotrypsin is different from that of trypsin: chymotrypsin cleaves a carboxy group at the C-terminal of an aromatic amino acid by hydrolysis. Also, native trypsin sometimes produces pseudo trypsin by autolysis, which exhibits a wide range of specificity including chymotrypsin-like activity. Using animal-derived trypsin as the protease, therefore, the substrate specificity may be lost by the influences of the mixed-in chymotrypsin and the autolysate of trypsin, whereby unintended peptide fragments may be produced, causing a problem of reduction in analytical accuracy.

It is difficult to completely remove chymotrypsin from animal-derived trypsin by purification. Therefore, animal-derived trypsin is mainly used for peeling of an adherent cell in cell culture. In order to use animal-derived trypsin as the protease for mass spectrometry, methods of improving the substrate specificity of trypsin by inactivation of chymotrypsin and suppression of autolysis of trypsin are known. For example, chymotrypsin mixed in trypsin is inactivated by treatment with N-tosyl-L-phenylalanine chloromethyl ketone (TPCK).

While the above chemical treatments are useful for improving the substrate specificity of animal-derived trypsin, they are likely to cause great reduction in enzyme activity and variations in activity among lots. For this reason, recombinant trypsin of mass spectrometry grade is mainly used for preparation of a sample for mass spectrometry for which high-accuracy quantitativity is required. Recombinant trypsin, which does not contain animal-derived impurities, has high substrate specificity and enzyme activity.

### PRIOR ART DOCUMENT

### PATENT DOCUMENT

Patent Document 1: WO2015/033479

### NON-PATENT DOCUMENT

Non-Patent Document 1: Iwamoto et. al, Analyst., 2014, 139(3), 576-80.
Non-Patent Document 2: Iwamoto et. al, Anal. Methods, 2015, 21, 9177-9183.
Non-Patent Document 3: Iwamoto et. al, Drug Metabolism and Pharmacokinetics, 2016, 31, 46-50.
Non-Patent Document 4: Iwamoto et. al, J. Chromatogr. B: Analyt. Technol. Biomed. Life Sci., 2016, 1023-1024, 9-16.
Non-Patent Document 5: Iwamoto et. al, Bioanalysis, 2016, 8(10), 1009-1020.
Non-Patent Document 6: Iwamoto et. al, Biol. Pharm. Bull., 2016, 39(7), 1187-1194.

### SUMMARY OF THE INVENTION

### PROBLEMS TO BE SOLVED BY THE INVENTION

As described above, recombinant trypsin exhibits high substrate specificity, and the usability thereof has been confirmed also in the nSMOL method in which an antibody is site-specifically cleaved. However, since recombinant trypsin is expressed with yeast, the production quantity thereof is small. Moreover, since recombinant trypsin needs to be purified by gel filtration chromatography after purification by affinity chromatography, the purification efficiency is low. For these reasons, it is difficult to secure a sufficient supply amount of recombinant trypsin, and moreover, recombinant trypsin is very expensive. Accordingly, recombinant trypsin is largely used for research purposes such as structural analysis of proteins, and industrial use thereof is limited. An objective of the present invention is providing a method for preparing a sample for mass spectrometry for performing quantitation of the concentration of an antibody in blood, etc. using animal-derived trypsin of which a sufficient supply amount can be secured and that is inexpensive.

### MEANS FOR SOLVING THE PROBLEMS

The present invention relates to a method for preparing peptide fragments in which an antibody, which includes a Fc domain immobilized in pores of a porous body, is brought into contact with a protease immobilized on the surfaces of microparticles, to site-specifically cleave the Fab domains of the antibody with the protease. The present invention further relates to a kit for preparing peptide fragments used for the above method. The kit of the present invention includes microparticles with a protease immobilized on their surfaces.

The protease immobilized on the surfaces of microparticles is animal-derived trypsin, which has chymotrypsin mixed therein, in which the chymotrypsin is inactivated. The trypsin immobilized on the surfaces of microparticles has a chemically-modified amino group in its lysine residue.

The chymotrypsin is irreversibly inactivated by allowing N-tosyl-L-phenylalanine chloromethyl ketone (TPCK) to bind to its histidine residue that is the active center, for example. The amino group in the lysine residue of the trypsin is reductive alkylated, for example.

The porous body is preferably capable of selectively immobilizing the Fc domain of an antibody in its pores. For example, a porous body coated with protein A and protein G is used.

The above-described protease can be prepared by altering animal-derived trypsin. The trypsin is altered by being subjected to treatment with a serine protease inhibitor and reductive alkylation, for example. The trypsin after the inactivation of chymotrypsin and the reductive alkylation is preferably purified by affinity chromatography.

The chymotrypsin is inactivated by allowing the animal-derived trypsin to react with the serine protease inhibitor. As the serine protease inhibitor, one that selectively deactivates chymotrypsin while not deactivating trypsin is preferable, and the above-described TPCK is preferably used.

By the reductive alkylation using an alkylating agent and a reducing agent, the amino group in the lysine residue of the trypsin is alkylated, suppressing autolysis of trypsin. The total amount of the alkylating agent used for the reductive alkylation is preferably 200 mole times or more that of the trypsin. The total amount of the reducing agent used for the reductive alkylation is also preferably 200 mole times or more that of trypsin. As the alkylating agent, aldehyde is preferable. By using formaldehyde as the alkylating agent, the amino group in the lysine residue of the trypsin is methylated.

### EFFECTS OF THE INVENTION

Since the altered trypsin used in the present invention is derived from an animal, it is inexpensive, and the supply amount thereof is easily secured, compared to the recombinant trypsin widely used for mass spectrometry. Also, in site-specific fragmentation of an antibody with the altered trypsin immobilized on the surfaces of microparticles, the altered trypsin exhibits substrate specificity and enzyme activity as high as or higher than those of the recombinant trypsin. Therefore, according to the present invention, the quantitation of the concentration of an antibody in a biological sample such as blood can be performed inexpensively and simply.

### BRIEF DESCRIPTION OF THE DRAWINGS

FIG. 1 is a conceptual diagram for illustrating the principle of site-specific cleaving of an antibody with protease.
FIG. 2 shows measurement results of the protein concentration and enzyme activity for fractions in chromatography.
FIG. 3 is a graph showing the production amounts of specific fragments of substrates.
FIG. 4A is a graph showing the evaluation results of chemical compatibility of an altered enzyme of an example.
FIG. 4B is a graph showing the evaluation results of chemical compatibility of an altered enzyme of an example.
FIG. 4C is a graph showing the evaluation results of chemical compatibility of an altered enzyme of an example.
FIG. 4D is a graph showing the evaluation results of chemical compatibility of an altered enzyme of an example.
FIG. 4E is a graph showing the evaluation results of chemical compatibility of an altered enzyme of an example.
FIG. 5 is a graph showing the production amounts of substrate-specific fragments by trypsin digestion of albumin, hemoglobin, and lactoglobulin.
FIG. 6A is a graph in which the relationship between the concentration of bevacizumab in plasma and the peak area in mass spectrometry of peptide fragments produced by site-specific fragmentation is plotted.
FIG. 6B is a graph in which the relationship between the concentration of bevacizumab in plasma and the peak area in mass spectrometry of peptide fragments produced by site-specific fragmentation is plotted.
FIG. 7 is a graph in which the relationship between the concentration of trastuzumab in plasma and the peak area in mass spectrometry of peptide fragments produced by site-specific fragmentation is plotted.
FIG. 8 is a graph in which the relationship between the concentration of nivolumab in plasma and the peak area in mass spectrometry of peptide fragments produced by site-specific fragmentation is plotted.
FIG. 9 is a graph showing the relationships of the use amount of an alkylating agent during alteration of trypsin with enzyme activity and with the production amount of fragments derived from CDRs of an antibody.

### MODE FOR CARRYING OUT THE INVENTION

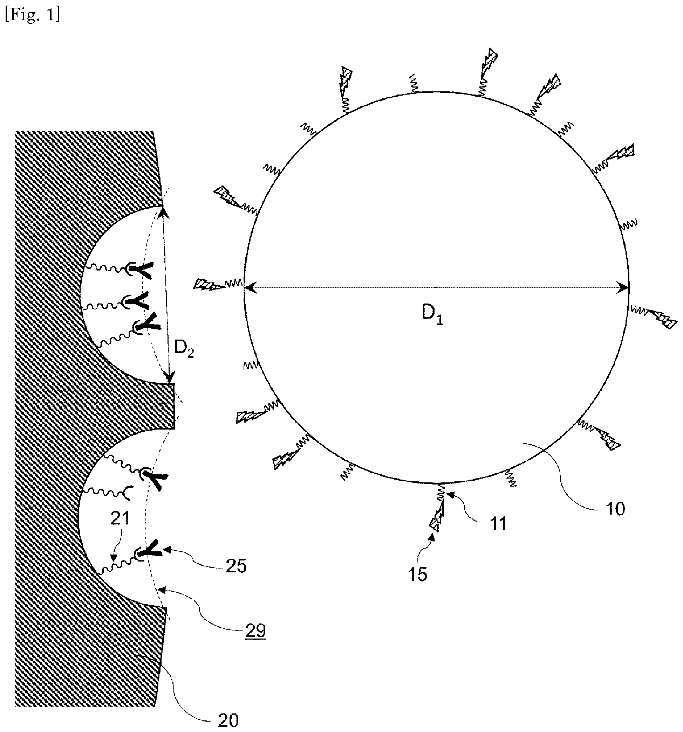

In the method of the present invention, a substrate immobilized in pores of a porous body is brought into contact with protease immobilized on the surfaces of microparticles, whereby the substrate is site-specifically cleaved with the protease to obtain peptide fragments. FIG. 1 is a conceptual diagram for illustrating the principle of site-specific cleaving of the substrate. Protease 15 is immobilized on the surface of a microparticle 10 (average particle diameter D₁). A porous body 20 has a number of pores 29 (average pore diameter D₂), and a substrate 25 is immobilized in such a pore 29.

### [Substrate]

The substrate 25 is an antibody, and the Fc domain of the antibody is preferably immobilized in the pore 29 of the porous body 20. With the Fc domain of the antibody immobilized in the porous body, the orientation of the antibody in the pore is controlled, permitting site-specific cleaving of Fab domains with the protease. The kind of the antibody is not particularly limited, but a monoclonal antibody is preferable. Among others, an antibody that requires quantitation of its concentration in a biological sample (e.g., blood) (e.g., a molecular targeted drug) is preferable. The antibody may be a human antibody, a humanized antibody, a chimeric antibody, and the like.

### [Porous body]

The material of the porous body 20 is not particularly limited as long as it has a number of pores 29. Activated carbon, a porous membrane, porous resin beads, metal particles, etc. may be used. Although semi-spherical pores are shown in FIG. 1, the shape of the pores is not particularly limited. A porous body having through pores, such as a porous membrane, can also be used.

The porous body is preferably capable of specifically binding with an antibody, particularly preferably capable of site-specifically binding with the Fc domain of the antibody. To achieve specific binding with an antibody, a linker 21 capable of specifically binding with the antibody is preferably immobilized on the surfaces of the pores of the porous body. Examples of the linker capable of site-specifically binding with the Fc domain of an antibody include protein A and protein G. That is, the porous body 20 is preferably coated with a molecule capable of specifically binding with an antibody, such as protein A and protein G. In particular, protein A is high in site specificity in binding with a Fc domain. For this reason, to enhance the site specificity of proteolysis, it is preferable to use a porous body coated with protein A.

The size of the pores 29 of the porous body 20 is not particularly limited. If the average pore diameter D₂ of the porous body 20 is smaller than the average particle diameter D₁ of the microparticle 10, the microparticle 10 is unable to access the deep part of each of the pores 29. Accordingly, the protease 15 immobilized on the surface of the microparticle 10 is unable to access the deep part of the pore 29.

In FIG. 1, the dotted line near the pore 29 represents the boundary of the region to which the protease 15 is accessible. In this way, the access of the protease to the Fc domain of the antibody bound to the surface of the pore is limited, and this relatively enhances the probability of access of the protease to the Fab domains of the antibody located apart from the binding site with the pore. As a result, the Fab domains of the antibody can be site-specifically cleaved with the protease to obtain peptide fragments.

To immobilize the Fc domain in the pore and site-specifically proteolyze the Fab domains, the average pore diameter of the porous body is preferably within the range between 30 nm and 150 nm, more preferably within the range between 40 nm and 120 nm, further preferably within the range between 50 nm and 100 nm.

### [Microparticles]

The microparticle 10 immobilizes the protease 15 on its surface and controls the access of the protease to the antibody 25 immobilized in the pore 29 of the porous body 20. To prevent the microparticle 10 from entering the deep part of the pore 29 of the porous body 20, the average particle diameter D₁ of the microparticle 10 is preferably larger than the average pore diameter D₂ of the porous body 20. The shape of the microparticle 10 is not particularly limited, but, from the standpoint of equalizing the region accessible for the protease to the pore 29 of the porous body 20, a spherical microparticle is preferable. Also, the microparticles 10 preferably have a uniform average particle diameter.

The average particle diameter D₁ of the microparticle 10 is more preferably 1.2 times or more, further preferably 1.5 times or more, particularly preferably 1.8 times or more, as large as the average pore diameter D₂ of the porous body 20. The average particle diameter D₁ of the microparticle 10 is preferably 100 nm or larger, more preferably 150 nm or larger. The upper limit of the average particle diameter D₁ of the microparticle 10 is not particularly limited, but is preferably 1 µm or less, more preferably 500 nm or less, from the standpoint of enhancing the efficiency of digestion with protease.

The material of the microparticle 10 is not particularly limited as long as it can immobilize protease on its surface, and metal, resin, and the like are used appropriately. As the microparticle 10, preferable is one capable of suppressing adsorption of any non-specific protein to its surface while selectively immobilizing protease to its surface. For example, a microparticle modified with a spacer 11 that specifically binds with protease is suitably used. As the spacer, one that can bind with protease and does not deactivate protease is preferable.

From the standpoint of controlling the access range of the protease 15 immobilized on the surface of the microparticle 10, the spacer 11 is preferably small in molecular diameter. The molecular diameter of the spacer is preferably 5 nm or less, more preferably 3 nm or less, further preferably 2 nm or less. The spacer 11 capable of immobilizing protease is preferably a non-protein, and preferably a molecule having a functional group such as an amino group, an amide group, an ester group, an epoxy group, a carboxyl group, biotin, avidin, and chelate.

### [Protease]

In the present invention, trypsin is used as the protease. Trypsin is small in molecular diameter and has its active site inside the molecule. Therefore, for the trypsin immobilized on the surface of the microparticle, the region in which the active site is accessible to the antibody as the substrate is limited, and therefore the selectivity of the cleaving site of the antibody is enhanced.

The trypsin used as the protease is animal-derived trypsin, which is derived from a pig or bovine pancreas, for example. While recombinant trypsin does not contain animal-derived impurities, trypsin derived from an animal's pancreas unavoidably has chymotrypsin mixed therein. In the present invention, chymotrypsin mixed in the trypsin used as the protease is inactivated.

Chymotrypsin can be inactivated by various kinds of serine protease inhibitors. Examples of the serine protease inhibitors usable for inactivation of chymotrypsin include phenylmethylsulfonyl fluoride (PMSF), 4-(2-aminoethyl)benzenesulfonyl fluoride (AEBSF), diisopropyl fluorophosphate (DFP), benzamidine, chymostatin, N-tosyl-L-lysyl chloromethyl ketone (TLCK), and N-tosyl-L-phenylalanine chloromethyl ketone (TPCK). Among others, TPCK is preferable because it can selectively and irreversibly inactivate chymotrypsin while maintaining the enzyme activity of trypsin.

Since TPCK irreversibly binds with a histidine residue included in the active center of chymotrypsin, chymotrypsin can be inactivated, while the activity of trypsin is maintained, with TPCK treatment. By mixing the animal-derived trypsin having chymotrypsin mixed therein with TPCK in a solution, the chymotrypsin is inactivated. The TPCK treatment may be performed at about 4°C for about 1 hour to 5 hours, for example.

The trypsin included in the protease used in the present invention has a chemically-modified amino group in its lysine residue. With an ε-amino group included in the lysine residue of the trypsin subjected to chemical modification, autolysis is suppressed, enhancing the enzyme activity and substrate specificity of the trypsin. As the chemical modification of the amino group, alkylation is preferable. For example, by reacting with aldehyde under the presence of a reducing agent, the amino acid in the lysine residue is reductive alkylated. The reducing agent used in the reductive alkylation is preferably one that is stable in an aqueous solution and capable of selectively reducing a Schiff base formed at the first stage of the reductive alkylation. Examples of a preferred reducing agent include sodium borohydride, sodium cyanoborohydride, dimethylamine borane, trimethylamine borane, and pyridine borane.

The amino group in the lysine residue is dialkylated under the presence of excessive amounts of aldehyde and a reducing agent relative to the amount of the lysine residue. For example, the amino group in the lysine residue is reductive dimethylated under the presence of excessive amounts of formaldehyde and a reducing agent. To suppress the autolysis of trypsin to enhance the substrate specificity, the use amount (total amount) of the alkylating agent (aldehyde) is preferably 200 mole times or more, more preferably 600 mole times or more, further preferably 1000 mole times or more, the amount of trypsin. The use amount of the reducing agent is preferably 200 mole times or more, more preferably 500 mole times or more, further preferably 800 mole times or more, the amount of trypsin. If the use amounts of the reducing agent and the alkylating agent are excessively large, reduction in enzyme activity and reduction in collection rate during purification may sometimes occur. Therefore, the use amounts of the reducing agent and the alkylating agent are preferably 20000 mole times or less, more preferably 10000 mole times or less, further preferably 5000 mole times or less, the amount of trypsin. The reducing agent and the alkylating agent may be added all at once or over a plurality of times.

After the inactivation of chymotrypsin and the modification of the amino group in the lysine residue of trypsin, purification is preferably performed. The purification method is not particularly limited, but purification by affinity chromatography using an affinity column capable of reversibly adsorbing trypsin is preferable. As a carrier for the affinity column, sepharose beads coupled with a serine protease inhibitor such as benzamidine, etc. are used. Fractions high in enzyme activity are selected from an eluate, to obtain trypsin high in substrate specificity and activity.

Altered trypsin obtained by subjecting animal-derived trypsin to the inactivation of chymotrypsin by the TPCK treatment, etc., to the modification such as reductive dimethylation, and to the purification by chromatography exhibits substrate specificity and enzyme activity as high as or higher than those of the recombinant trypsin, as will be described in the examples to follow. Also, the altered trypsin easily responds to increase in supply amount and is inexpensive, compared to the recombinant trypsin.

In the present invention, in addition to the altered animal-derived trypsin, another type of protease may be used. For example, lysyl endopeptidase that selectively hydrolyzes the carboxy group at the C-terminal of the lysine residue may be used additionally. In the case of using a protease other than trypsin, the amount of trypsin in the entire protease is preferably 90% or more.

### [Sample preparation method]

In the method for preparing peptide fragments according to the present invention, an antibody included in a sample such as blood is immobilized on a porous body, and then the porous body on which the antibody is immobilized is brought into contact with microparticles on which protease is immobilized, whereby the antibody is site-specifically cleaved with the protease to produce peptide fragments.

The method for immobilizing an antibody as the substrate in pores of the porous body is not particularly limited. When the porous body is coated with a molecule capable of specifically binding with an antibody, such as protein A and protein G, a suspension of the porous body may be mixed with a solution including the antibody, whereby the antibody can be easily immobilized in pores of the porous body.

An example of the solution including an antibody is a sample derived from a living body, such as blood. The living body-derived sample contains a wide variety of impurities in addition to the antibody. The porous body and the living body-derived sample as a specimen are mixed together, and cleaned in the state where the antibody is selectively immobilized on the porous body, to remove impurity components non-selectively adhering to the porous body. When the impurity components tend to cohere and strongly adhere to the porous body, the impurity components are preferably denatured using a surfactant, etc. and dissolved in a liquid. After the treatment with the surfactant, the mixture is preferably cleaned with a liquid containing no surfactant to remove the surfactant adhering to the porous body.

The method for immobilizing protease on the surfaces of microparticles is not particularly limited, but an appropriate method can be adopted depending on the properties of the protease and the microparticles (or spacer molecules modifying the surfaces of the microparticles). For example, when trypsin is to be immobilized on the spacer-modified surfaces of the microparticles, a suspension of the microparticles and a solution containing trypsin are mixed together, to achieve immobilization of the protease on the surfaces of the microparticles. After the immobilization of the protease on the surfaces of the microparticles, active portions unbound with the protease on the surfaces of the microparticles may be inactivated with chemical modification, and the like.

The protease may be presented in the previously immobilized state on the microparticles, or may be immobilized on the surfaces of the microparticles immediately before use. The microparticles on which the protease is immobilized is preferably presented as a suspension.

The microparticles on which the altered trypsin is immobilized may be presented as part of a kit for preparing peptide fragments. Trypsin can retain its activity even when being immobilized on the surfaces of the microparticles. Therefore, having, as a component of the kit, the protease immobilized on the surfaces of the microparticles, the operation for preparing peptide fragments can be simplified. The kit for preparing peptide fragments may include, in addition to the microparticles on which the altered trypsin is immobilized, a porous body that can selectively immobilize the Fc domain of the antibody in its pores. As the porous body that can selectively immobilize the Fc domain of the antibody in its pores, one coated with protein A, protein G, etc. is preferable as described above. The kit for preparing peptide fragments may further include a container, a solution, etc. used for immobilization of the antibody in a specimen on the porous body, for reaction, etc.

The porous body on which the antibody is immobilized and the microparticles on which protease is immobilized are mixed in a liquid, whereby site-specific proteolysis of the antibody is performed. The reaction conditions are not particularly limited, but conditions similar to those for general proteolysis can be adopted appropriately. For example, incubation is preferably performed in a buffer solution adjusted to a pH near the optimum pH for trypsin (about pH 7 to 9) normally at a temperature of about 35 to 60°C for about 3 hours to 30 hours.

The peptide fragments produced by the reaction with the protease are liberated in the solution. Since access of the protease to the Fc domain of the antibody, which is a binding site to the porous body, is limited, the Fab domains of the antibody including the complementarity determining regions (CDRs) are site-specifically cleaved with the protease. Therefore, in the solution having the liberated peptide fragments, peptide fragments including amino acid sequences of the CDRs, which are important for identification of the antibody, are contained at high concentration.

### [Quantitation of antibody concentration by mass spectrometry]

The sample containing the peptide fragments obtained as described above is analyzed by chromatography and mass spectrometry, whereby the antibody can be identified and quantitated. According to the present invention, in which the Fab domains of the antibody are site-specifically cleaved with trypsin, the number of kinds of peptide fragments contained in the sample is small, and the peptide fragments including amino acid sequences of the CDRs are contained at high concentration. Therefore, the analytical conditions for mass spectrometry, etc. can be easily set.

In order to further ensure the separation of the peptide fragments to enhance the analytical accuracy, the sample may be separated and concentrated by liquid chromatography (LC), solid phase extraction (SPE), etc. before being subjected to the mass spectrometry.

For identification of the antibody based on the results of mass spectrometry, existing databases may be used. For quantitation of the antibody concentration, multiple reaction monitoring (MRM) using LC/MS/MS is suitable. In quantitation of the content (concentration) of the antibody in a specimen such as blood, the amount of the antibody can be calculated based on the peak area and peak strength of ions derived from the peptide fragments. For example, the concentration of the peptide fragments in a specimen is calculated by creating association between a previously-determined calibration curve and the peak area, association between a peak area derived from an internal standard added to the sample and a peak area derived from the sample, etc. Based on this, the amount and concentration of the antibody in a specimen such as blood are calculated.

The altered trypsin described above has excellent substrate specificity similar to that of the recombinant trypsin widely used for mass spectrometry, and is excellent in the site-specificity of a cleaving site of an antibody. Therefore, the altered trypsin is high in reproducibility of mass spectrometry and can be used for quantitation of the concentration of an antibody drug in a specimen such as blood.

The method of the present invention is easy to operate, can secure reproducibility and quantitativity, and can be automated. Further, since the protease used for sample preparation can be supplied stably and is inexpensive, the method is high in industrial utility value. The method of the present invention is applicable to fundamental researches such as pharmacokinetic analysis, interactive analysis using antigen-antibody reaction, various interactome analyses, and identification of immunoprecipitated proteins. Other applications to sequencing analysis of biomolecular drugs such as antibody drugs, quality assurance, confirmation of identity of generic drugs, etc. are also expected.

### EXAMPLES

The present invention will be described more concretely by presenting an example as follows. It is however to be noted that the present invention is not limited to the following example. In the following description, the symbol % represents % by weight unless otherwise specified.

### [Alteration of trypsin and chromatography purification]

### (TPCK treatment)

Pig pancreas trypsin (SIGMA ALDRICH, T0303) was dissolved in a 50 mM sodium phosphate buffer solution (pH 7.4) to prepare a solution of 1 mg/mL (about 40 µM). To this solution, a DMSO solution (40 mM) of N-tosyl-L-phenylalanine chloromethyl ketone (TPCK) was added so that the TPCK concentration be 100 µM, and the resultant solution was gently stirred at 4°C for 2 hours.

### (Reductive dimethylation)

To the TPCK-treated trypsin solution, 1M dimethylamine borane and 1M formaldehyde were added to have 20 µmol of dimethylamine borane and 40 µmol of formaldehyde relative to 1 mg of trypsin, and the resultant solution was gently stirred at 4°C for 1.5 hours. The same amounts of dimethylamine borane and formaldehyde were further added and gently stirred at 4°C for 1.5 hours. Thereafter, 10 µmol of dimethylamine borane was added relative to 1 mg of trypsin, and the resultant solution was gently stirred at 4°C overnight. In this treatment, the amounts of formaldehyde about 2000 mole times, and dimethylamine borane about 1250 mole times, that of trypsin were used.

### (Chromatography purification)

A 50 mM sodium phosphate buffer solution (pH 7.4) of an amount 10 times as large as the column capacity was allowed to flow through a 2mL-capacity benzamidine sepharose column (GE Healthcare HiTrap Benzamidine FF Column; two 1-mL columns were coupled) to achieve equilibration, and then the above-described treated trypsin solution (trypsin amount: 25 mg) was allowed to flow through the column to immobilize trypsin on the column. A 50 mM sodium phosphate buffer solution (pH 7.4) containing a sodium chloride concentration of 150 mM was allowed to flow through the column by an amount 10 times as large as the column capacity, to clean the column, and then a 10 mM sodium phosphate buffer solution (pH 7.4) of an amount 4 times as large as the column capacity was allowed to flow through the column to demineralize the column. Thereafter, elution was performed with 10 mL of 10 mM hydrochloric acid to collect first 0.5 mL of the eluate as Fraction No. 1 and subsequent every 1 mL of the eluate as each fraction (Fraction Nos. 2 to 10).

### (Quantitation of protein concentration)

From each of the above fractions, 50 µL was taken, to which a 1 M tris hydrochloride buffer solution (pH 8.0) was added to quantitate the protein concentration with Micro BCA™ Protein Assay Kit.

### (Enzyme activity of trypsin)

N-α-benzoyl-DL-arginine-p-nitroanilide hydrochloride (BANA) as the substrate was added to a 50 mM tris hydrochloride buffer solution (pH 8.0) so as to have a concentration of 50 µL/mL. To this solution, each fraction of the eluate was added to have a trypsin concentration of 10 µL/mL. While the resultant solution was gently stirred at 37°C, the absorbance thereof at 405 nm was measured every fixed hour, to calculate the activity of the trypsin from the change of the absorbance with time (the rate of production of p-nitroaniline due to decomposition of the substrate).

### (Production amount of specific fragments)

Using albumin (Alb), hemoglobin (Hb), and lactoglobulin (Lac) as substrates, the above Fraction Nos. 1 to 10 were added to the substrates and subjected to digestion reaction at 37°C overnight. The solutions after the digestion reaction were subjected to MS/MS analysis, and by analysis with a database (Mascot server), the production amounts of specific fragments of the substrates were determined. Using trypsin subjected to only TPCK treatment and not subjected to reductive dimethylation and a commercially available product of recombinant trypsin (Promega Trypsin Gold) as enzymes, the production amounts of specific fragments of the substrates were determined in a manner similar to the above.

### (Evaluation results and determination of collected fraction)

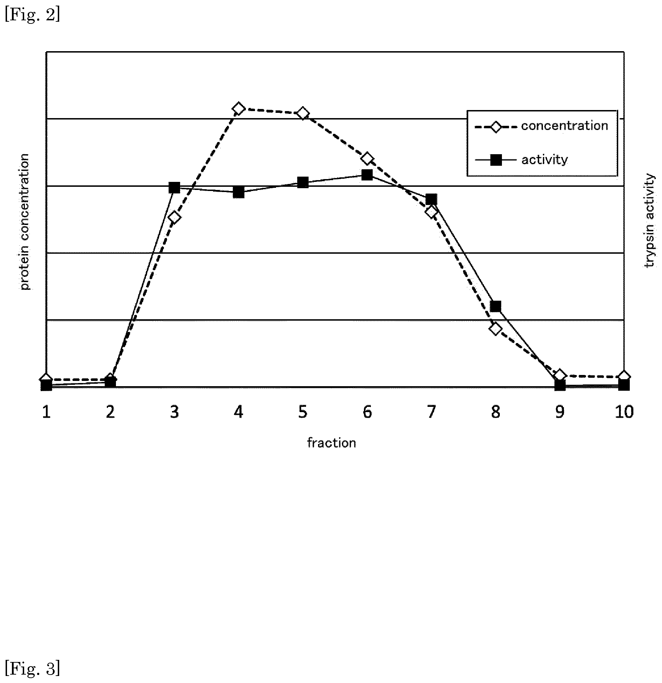

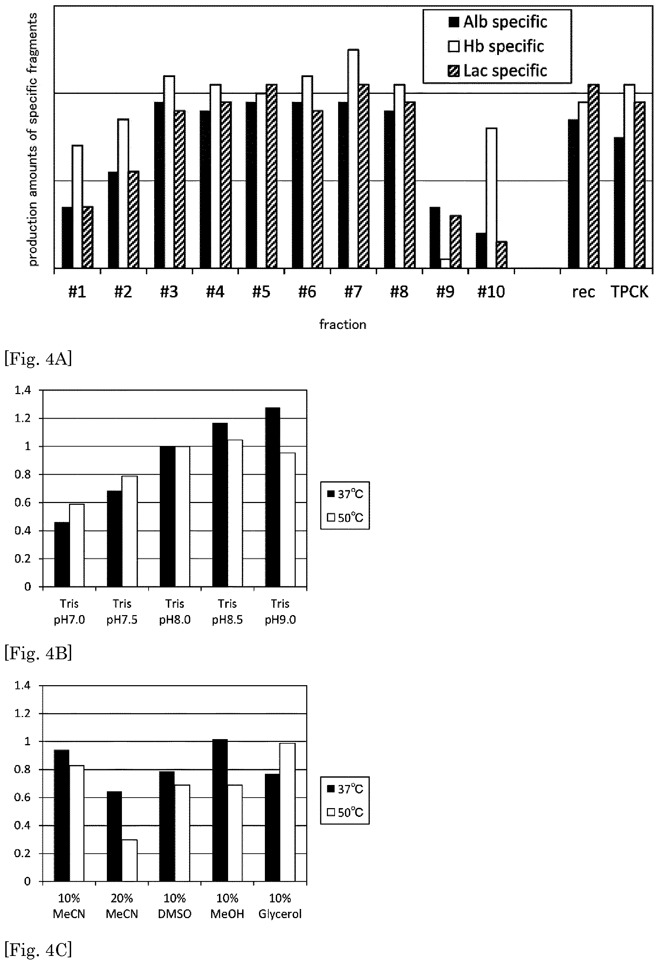

FIG. 2 shows the measurement results of the protein concentration and enzyme activity for Fraction Nos. 1 to 10. FIG. 3 shows the production amounts of specific fragments obtained when the substrates (Alb, Hb, and Lac) were digested using Fraction Nos. 1 to 10, the recombinant trypsin (rec), and the trypsin subjected to only TPCK treatment (TPCK).

As shown in FIG. 2, Fraction Nos. 3 to 7 were high in protein concentration and exhibited roughly equal enzyme activities. As shown in FIG. 3, Fraction Nos. 3 to 8 were comparable, in the production amount of substrate-specific fragments, to the commercially available recombinant trypsin. Based on these results, Fraction Nos. 3 to 7 were determined to be collected fractions. Using these collected fractions (hereinafter sometimes referred to as the "altered trypsin"), the following evaluation was performed.

### [Evaluation of enzyme activity]

### (Comparison in enzyme activity with recombinant trypsin)

The enzyme activities of the altered trypsin and the commercially available recombinant trypsin were measured using N-α-benzoyl-DL-arginine-p-nitroanilide hydrochloride as the substrate. As a result, the altered trypsin exhibited enzyme activity about twice as high as the recombinant trypsin.

### (Chemical compatibility)

The enzyme activity of the altered trypsin was measured while the pH of a tris hydrochloride buffer solution being changed in the range of 7.0 to 9.0 and at reaction temperatures of 37°C and 50°C. FIG. 4A shows a graph of the enzyme activities at 37°C and 50°C normalized with respect to the enzyme activities at pH 8.0 that are set to 1.

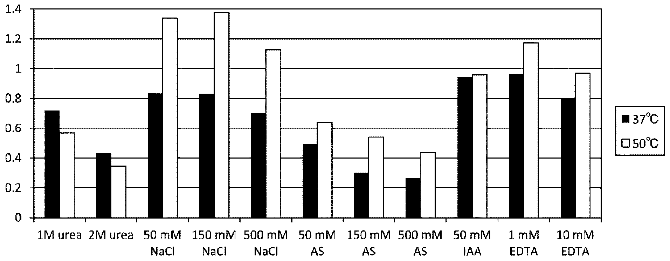

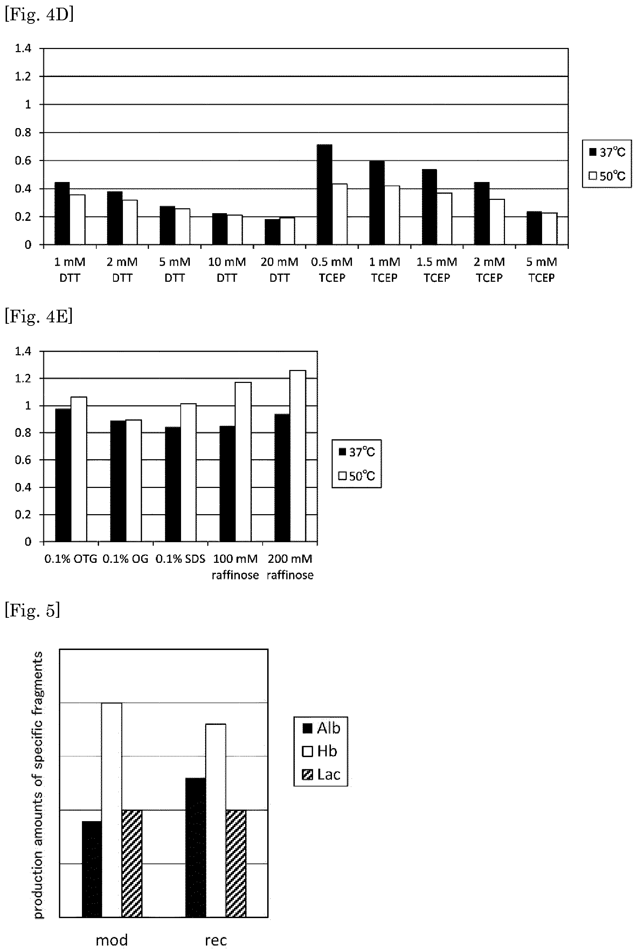

FIG. 4B shows the measurement results of enzyme activities in solutions of acetonitrile (10% and 20%), DMSO (10%), methanol (10%), and glycerol (10%). FIG. 4C shows the measurement results of enzyme activities in solutions of urea (1M and 2M), sodium chloride (50 mM, 150 mM, and 500 mM), ammonium sulfate (50 mM, 150 mM, and 500 mM), indole-3-acetic acid (50 mM), and ethylenediaminetetraacetic acid (1 mM and 10 mM). FIG. 4D shows the measurement results of enzyme activities in solutions of dithiothreitol (1 mM, 2 mM, 5 mM, 10 mM, and 20 mM), and tris(2-carboxymethyl)phosphine (0.5 mM, 1 mM, 1.5 mM, 2 mM, and 5 mM). FIG. 4E shows the measurement results of enzyme activities in solutions of n-octyl-β-D-thioglucoside (0.1%), n-octyl-β-D-glucoside (0.1%), sodium dodecyl sulfate (0.1%), and raffinose (100 mM and 200 mM). The enzyme activities in FIGS. 4B to 4E are shown as values normalized with respect to the enzyme activities in the tris hydrochloride buffer solution at pH 8.0 at 37°C and 50°C (see FIG. 4A) that are set to 1.

### [Immobilization of trypsin on microparticle surface]

An isopropanol suspension, 50 µL, of poly(glycidyl methacrylate)-coated ferrite nanoparticles (FG beads manufactured by Tamagawa Seiki Co., Ltd., particle diameter: about 200 nm) was centrifuged at 4°C to spin down microparticles. After removal of the supernatant liquid, the remainder was cleaned with methanol. A solution containing 50 µg of the altered trypsin was added to the microparticles to suspend the microparticles. The suspension of the microparticles is stirred at 4°C for 30 minutes and centrifuged at 4°C to spin down the microparticles, and the supernatant liquid was removed. Cleaning with a 50 mM tris hydrochloride buffer solution (pH 8.0) and centrifugation were repeated twice, and then the microparticles were suspended in a tris hydrochloride buffer solution (pH 8.0), to obtain a suspension of trypsin immobilized on the surfaces of the microparticles at a concentration of 1 µg/µL.

Using the commercially available recombinant trypsin in place of the altered trypsin, a suspension of microparticles on the surfaces of which the recombinant trypsin was immobilized was prepared.

### (Comparison of enzyme activity)

The enzyme activities of the altered trypsin and the recombinant trypsin immobilized on the surfaces of the microparticles were measured using N-α-benzoyl-DL-arginine-p-nitroanilide hydrochloride as the substrate. As a result, the altered trypsin immobilized on the surfaces of the microparticles exhibited enzyme activity about 4 times as high as the recombinant trypsin immobilized on the surfaces of the microparticles.

### (Production amount of specific fragments)

Using albumin (Alb), hemoglobin (Hb), and lactoglobulin (Lac) as substrates, the trypsin-immobilized microparticles were added to the substrates and subjected to digestion reaction at 37°C overnight. By database analysis of MS/MS analysis results, the production amounts of specific fragments of the substrates were determined. The results are shown in FIG. 5. The altered trypsin (mod) immobilized on the microparticles exhibited the production amounts of specific fragments comparable to those of the recombinant trypsin (rec).

### [Fragmentation of antibody by nSMOL method]

Monoclonal antibodies of predetermined concentrations (3.7, 11.1, 33.3, 100, and 300 µg/mL) were added to human plasma. As the antibodies, bevacizumab, trastuzumab, and nivolumab were used. The antibody-added plasma, 10 µL, was diluted to 10 times with phosphate buffered saline (PBS) containing 0.1% of octylthioglycoside.

A suspension, 25 µL, of a porous body coated with protein A (TOYOPEARL AF-rProtein A HC-650F manufactured by Tosoh Corporation, particle diameter: 30 to 60 µm, porosity: 86%, resin concentration: 50 wt%, IgG static adsorption amount: 80 g/L) was added to each of the above samples. The resultant suspension was gently stirred at room temperature for 15 minutes, to immobilize the antibody in plasma on the protein A-coated resin. The suspension was cleaned with PBS containing 0.1% of octylthioglycoside, and then cleaned with PBS to remove octylthioglycoside.

To the antibody-immobilized protein A-coated resin, 75 µL of 25 mM tris hydrochloride (pH 8.0) and 10 µL of a suspension of trypsin-immobilized microparticles were added, and left to reaction while being gently stirred at 50°C for 5 hours. Formic acid was added to stop the reaction so that the final concentration be 0.5%. The resin was removed by centrifugal filtration, and the collected filtrate was analyzed with LC-MS/MS (LCMS-8050 manufactured by Shimadzu Corporation). The analysis conditions were as follows.

### <LC conditions>

Mobile phase A: 0.1% formic acid aqueous solution
Mobile phase B: 0.1% formic acid, acetonitrile solution
Autosampler cleaning: ultrapure water
Column: Shim-Pack GISS (inner diameter: 2.1 mm, column length: 50 mm, particle diameter: 1.9 µm, pore diameter: 20 nm)
Flow rate: 0.4 mL/minute
Column oven temperature: 50°C
Sample cooler temperature: 5°C

### <MS conditions>

Interface voltage: 1.5 kV
Nebulizer gas flow rate: 3 L/minute
Heating gas flow rate: 10 L/minute
Drying gas flow rate: 10 L/minute
Interface temperature: 350°C
DL temperature: 250°C
Heat block temperature: 400°C

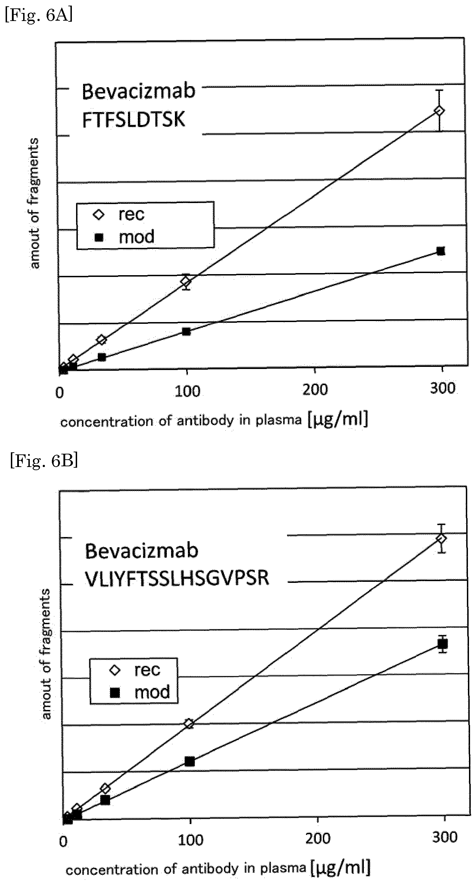

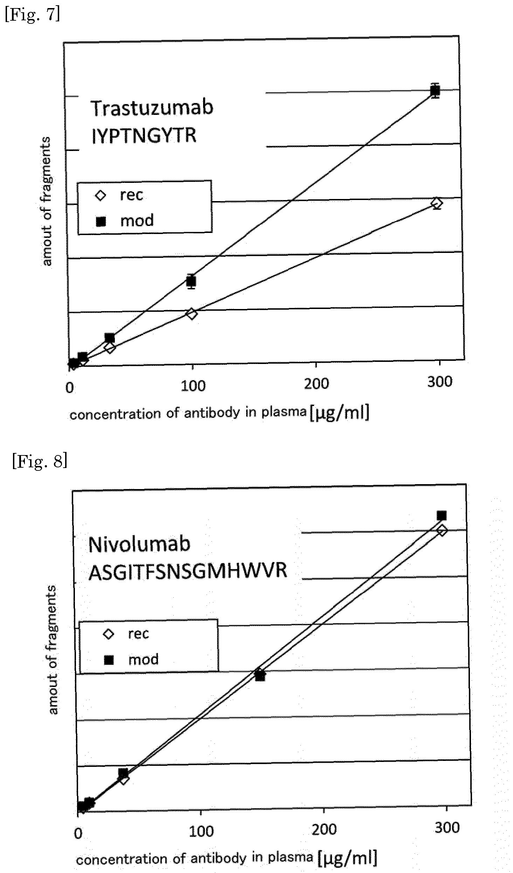

FIGS. 6A and 6B plot the relationships between the concentration of bevacizumab in plasma and the detected amounts (peak areas in mass spectrometry) of trypsin-digested peptide fragments (FTFSLDTSK and VLIYFTSSLHSGVPSR) derived from CDRs of bevacizumab. FIG. 7 plots the relationship between the concentration of trastuzumab in plasma and the detected amount of trypsin-digested peptide fragments (IYPTNGYTR) derived from CDRs of trastuzumab. FIG. 8 plots the relationship between the concentration of nivolumab in plasma and the detected amount of trypsin-digested peptide fragments (ASGITFSNSGMHWVR) derived from CDRs of nivolumab.

In all of the three kinds of antibody drugs (four kinds of CDR-derived peptide fragments), using the altered trypsin, high linearity was exhibited between the concentration of the antibody drug in plasma and the detected amount of peptide fragments, as in the case of using the recombinant trypsin. From these results, it is found that the concentration of an antibody drug in plasma can be quantitated using the altered trypsin, as in the case of using the recombinant trypsin.

### [Relationship between enzyme activity and production amount of peptide fragment by nSMOL method]

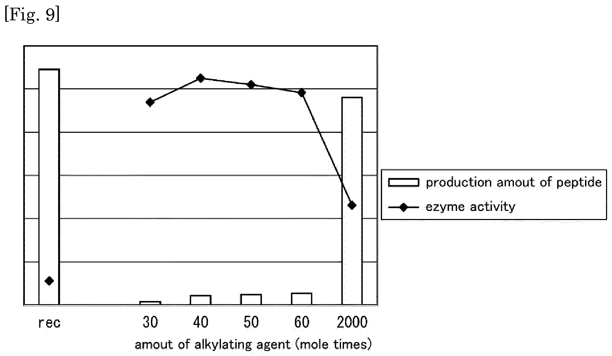

In the above example, the amount of the alkylating agent (formaldehyde) about 2000 mole times that of trypsin was used in the reductive dimethylation for trypsin alteration. This preparation was made so that the production amount of CDR-derived specific peptide fragments be maximum in the fragmentation of the antibody by the nSMOL method. FIG. 9 is a graph showing the relationships of the use amount of the alkylating agent with the enzyme activity of protease immobilized on microparticles (obtained using BANA as the substrate) and with the production amount (peak area of mass spectrometry) of fragments derived from CDRs of the antibody (FTFSLDTSK fragments of bevacizumab). In FIG. 9, the evaluation results of the commercially available recombinant trypsin (rec) are also shown.

When the use amount of the reductive alkylating agent was 30 times, 40 times, 50 times, and 60 times that of trypsin, the altered trypsin immobilized on microparticles exhibited enzyme activities about 10 times that of the recombinant trypsin immobilized on microparticles, but the production amounts of CDR-derived specific peptide fragments by the nSMOL method were 1/20 or less of that of the recombinant trypsin. On the contrary, when the use amount of the reductive alkylating agent was 2000 mole times that of trypsin, the enzyme activity of the altered trypsin immobilized on microparticles was about 4 times that of the recombinant trypsin immobilized on microparticles, and the production amount of CDR-derived specific peptide fragments by the nSMOL method was comparable to that of the recombinant trypsin.

From the above results, it is found that the enzyme activity does not necessarily correlate with the production amount of peptide fragments by site-specific cleaving of an antibody. It is also found that an altered trypsin capable of site-specifically cleaving an antibody can be obtained by reductive alkylation using an excessive amount of a reductive alkylating agent relative to trypsin.

## Claims

1. A method for preparing peptide fragments, the method comprising bringing an antibody, which includes a Fc domain immobilized in pores of a porous body, into contact with protease immobilized on surfaces of microparticles, to site-specifically cleave Fab domains of the antibody with the protease, wherein
the protease is animal-derived trypsin, which has chymotrypsin mixed therein, and
the chymotrypsin is inactivated, and an amino group in a lysine residue of the trypsin is chemically modified.

2. The method for preparing peptide fragments according to claim 1, wherein the chymotrypsin is inactivated by allowing N-tosyl-L-phenylalanine chloromethyl ketone to bind to a histidine residue.

3. The method for preparing peptide fragments according to claim 1, wherein the amino group in the lysine residue of the trypsin is dimethylated.

4. The method for preparing peptide fragments according to claim 1, wherein an average particle diameter of the microparticles is larger than an average pore diameter of the porous body.

5. The method for preparing peptide fragments according to claim 1, wherein an average pore diameter of the porous body is within the range between 30 and 150 nm, and an average particle diameter of the microparticles is 100 nm or larger.

6. The method for preparing peptide fragments according to claim 1, wherein the porous body has a surface coated with protein A or protein G.

7. A kit for preparing peptide fragments for use in the method according to any one of claims 1 to 6, wherein
the kit comprises microparticles on surfaces of which protease is immobilized,
the protease is animal-derived trypsin having chymotrypsin mixed therein, and
the chymotrypsin is inactivated, and an amino group in a lysine residue of the trypsin is reductive alkylated.

8. The kit for preparing peptide fragments according to claim 7, further comprising a porous body capable of selectively immobilizing a Fc domain of an antibody in pores.

9. A method for preparing protease for use in the method according to any one of claims 1 to 6, comprising the steps of:
inactivating chymotrypsin mixed in animal-derived trypsin by allowing the trypsin to react with a serine protease inhibitor; and
alkylating an amino group in a lysine residue of the trypsin by reductive alkylation using an alkylating agent and a reducing agent,
wherein the total amount of the alkylating agent used for the reductive alkylation is 200 mole times or more that of the trypsin.

10. The method for preparing protease according to claim 9, wherein the serine protease inhibitor is N-tosyl-L-phenylalanine chloromethyl ketone.

11. The method for preparing protease according to claim 9, wherein the alkylating agent is formaldehyde.

12. The method for preparing protease according to claim 9, wherein purification by affinity chromatography is performed after the inactivation of chymotrypsin and the alkylation of an amino group in a lysine residue of the trypsin.
